# EUROPEAN PATENT SPECIFICATION

(11) **EP 0 349 066 B1**
(45) Date of publication and mention of the grant of the patent: **26.04.1995**
(21) Application number: 89201651.0
(22) Date of filing: 22.06.1989
(51) Int. Cl.: C07D 295/08

(54) **Preparation of enantiomers of dropropizine**
Herstellung von Enantiomeren des Dropropizins
Préparation des énantiomères de la dropropizine

(30) Priority: 25.06.1988 NL 8801622
(43) Date of publication of application: 03.01.1990
(73) Proprietor: DSM N.V., 6411 TE Heerlen (NL)
(72) Inventor: Van Iersel, Jacobus Theodorus Matheus, NL-5925 AJ Venlo (NL); Elferink, Vincentius Henricus Maria, NL-7131 CT Lichtenvoorde (NL)

(56) References cited:
- EP-A- 0 147 847
- BE-A- 601 394
- JOURNAL OF THE AMERICAN CHEMICAL SOCIETY, vol. 106, no. 23, 14th November 1984,pages 7250-7251, American Chemical Society, Washington, D.C., US; "W.E. LADNERet al.: "Lipase-catalyzed hydrolysis as a route to esters of chiral epoxyalcohols"
- J. Jacques et al. "Enantiomers, Racemates and Resolutions", J. Wiley & Sons, Inc. (1981), Part 2, Chapter 7.6, pp. 423-8
- J. ORG. CHEM. 1986, vol. 51, pp. 5413-5415 S.Y. Ko et al "In Situ Opening of Epoxy Alcohols: A Convenient Alternative to the Isolation of Unstable Epoxy Alcohols"

## Description

The invention relates to the preparation of enantiomers of dropropizine. Dropropizine is the common name for 3-(4-phenyl-1-piperazinyl)-1,2-propanediol. For brevity's sake the common name 'dropropizine' will be used in the present description.

Dropropizine is used in medicine as an antitussive.

The preparation of the individual enantiomers of dropropizine is known from EP-A-147.847. This patent publication also mentions that the laevorotatory enantiomer ((-)-dropropizine) has a more selective effect and presents fewer undesired side-effects than the dextrorotatory enantiomer. It is therefore preferable to use the laevorotatory enantiomer, (-)-dropropizine, as an antitussive in medicine. This enantiomer must be as free as possible from the (+)-enantiomer.

Although EP-A-147.847 describes preparation processes of the enantiomers of dropropizine, the processes described are laborious and econimically unattractive. Recovering the desired enantiomer from racemic dropropizine is an uneconomical process because half of the raw and auxiliary materials, energy, etc. is used to prepare the undesired enantiomer, which has to be separated from the desired enantiomer and is then discarded or has to be racemized for re-use. Moreover, the separation of enantiomers always involves losses, which means a further reduction of the already small yield of the desired enantiomer. It is therefore far more preferable to prepare, where possible, substantially only the desired enantiomer. Such a preparation process directed towards the individual enantiomers has already been described in EP-A-147.847, but although this process does not present the aforementioned disadvantages, it is laborious and therefore rather uneconomical. According to that process, (-)-dropropizine is prepared from (+)-1,2-isopropylidene-snglycerol. Two processes are described for the preparation of 1,2-isopropylidene-sn-glycerol, which each consist of a number of steps. The (+)-1,2-isopropylidene-sn-glycerol must be converted into the corresponding tosylate; this tosylate must be deketalated and then the (-)-glycerol tosylate thus obtained must be coupled with a double amount of 1-phenylpiperazine to form (-)-dropropizine. This means that the synthesis of the (+)-1,2-propylidene-sn-glycerol and also the synthesis of dropropizine obtained from (+)-1,2-isopropylidene-snglycerol are both multi-step reactions. Another disadvantage is that the coupling of glycerol tosylate with a double amount of 1-phenylpiperazine is carried out in benzene. For considerations of health, benzene is an undesired solvent. The use of an excess of 1-phenylpiperazine is uneconomic.

The processes according to EP-A-147.847 are laborious and expensive and therefore there is a need for a simpler and/or cheaper process.

In Ko et al. J. Org. Chem. 1986, 51, 5413-5415 is disclosed the preparation of structurally similar compounds having an open ring structure obtained in a cleavage of the glycidol epoxide ring. However, from the experimental section it is clear that no e.e. enrichment is obtained by recrystallization of the compound obtained by in situ opening of glycidol by 1-naphtol.

A simple, non-stereospecific process for the preparation of dropropizine by coupling 1-phenylpiperazine with glycidol (1,2-epoxy-3-hydroxypropane) is known from BE-601.394. The enantiomers of glycidol were known long before BE-601.394 was filed, as appears from the article by John C. Sowden and Hermann O.L. Fischer in J. Am. Chem. Soc. (1942) 64, 1291 and the literature references therein. However, those known preparation processes for the enantiomers of glycidol are laborious. Once the importance of the individual enantiomers of dropropizine was realized, (-) and (+)-dropropizine were not prepared by coupling a glycidol enantiomer with 1-phenylpiperazine, but according to a different process as described in EP-A-147.847.

According to a first aspect of the invention it was found that enantiomers of dropropizine can be easily and economically prepared by coupling, in a manner known per se, 1-phenylpiperazine with glycidol containing an excess of one of the enantiomers of glycidol and by then crystallizing the dropropizine thus obtained, which contains an excess of one of the enantiomers of dropropizine, from a suitable solvent. Enantiomeric excess is expressed as the difference in weight percentage between the two enantiomers. The weight percentage is relative to the combined amount of the enantiomers. According to the invention it is easy to prepare dropropizine of great optical or enantiomeric purity in this manner.

Most surprisingly, it has now been found that dropropizine with a greatly increased enantiomeric excess can be obtained from dropropizine with a small enantiomeric excess by recrystallization from a suitable solvent. Depending on the original enantiomeric excess, one to two recrystallizations are usually sufficient to obtain an enantiomeric excess of about 99% or more.

The enantiomeric excess of the glycidol that is coupled to the 1-phenylpiperazine may vary within wide limits. Generally the enantiomeric excess will be at least 30%. There are no upper limits to the enantiomeric excess of the glycidol and it will be obvious that optically pure glycidol may be used to prepare optically pure dropropizine. As already mentioned above, the known preparation processes for optically pure glycidol are laborious. The preparation of glycidol that is however not optically pure but does contain an enantiomeric excess of one of the enantiomers, which enantiomeric excess may vary from about 30% to 95% and sometimes, even more, appears, as will be pointed out below, to be much simpler than the known preparation process of optically pure glycidol. It now appears to be possible to use such a glycidol with advantage for the synthesis of dropropizine because the dropropizine thus obtainable can easily be purified into optically pure or virtually pure dropropizine via one or two recrystallizations.

It will be clear that the process according to the invention may be used for the preparation of any enantiomer of dropropizine. With a view to the use of dropropizine for medicinal purposes e.g. as an antitussive, the preparation process will of course be directed towards (-)-dropropizine, but the preparation can in the same manner be directed towards (+)-dropropizine if so desired for whatever reason.

For the preparation of (-)-dropropizine (+)-glycidol must be coupled with 1-phenylpiperazine and for the preparation of (+)-dropropizine (-)-glycidol must be coupled.

According to a second aspect of the invention it has been found that dropropizine containing an excess of one of the enantiomers can be prepared by coupling 1-phenylpiperazine with glycidol containing an excess of one of the enantiomers in a known manner, which glycidol has been obtained by enantionselective enzymatic hydrolysis of a glycidyl ester.

The enantionselective enzymatic hydrolysis of, for example, glycidyl esters has recently been described by W.E. Ladner and G.M. Whitesides in J. Am Chem. Soc. (1984) 106 7250. Apparently the hydrolysis of the enantiomers proceeds at different rates. Partial hydrolysis of the d,l-ester results in glycidol with an excess of one of the enantiomers and the remaining, unhydrolysed part of the ester contains an excess of the ester of the other glycidol enantiomer. After separation of the glycidol formed in the enantionselective enzymatic hydrolysis from the remaining ester, the ester can be hydrolyzed or reesterified with lower alcohols in any suitable manner, both enzymatically and by basic or acid hydrolysis. Of course, this results in glycidol with an excess of the other enantiomer. In this manner racemic glycidol can be separated, via an ester and the enantioselective enzymatic hydrolysis thereof, into two fractions, one of which contains an excess of the (+)-enantiomer and the other an excess of the (-)-enantiomer of glycidol. Of course, both the glycidol formed in the first case and the glycidol obtained in the second case can be coupled with 1-phenylpiperazine and by working up the dropropizine thus obtained either of the two enantiomers can be prepared. For medical use as an antitussive, (-)-dropropizine is of importance. According to the invention (-)-dropropizine is prepared by coupling 1-phenylpiperazine with glycidol containing an excess of the (+)-enantiomer. In the enantiospecific enzymatic hydrolysis the glycidol usually contains an excess of the (+)-enantiomer and the remaining ester an excess of the (-)-enantiomer. The glycidol can be separated from the ester and can be used for the coupling with 1-phenylpiperazine. If an enzyme is used with which glycidol with an excess of (-)-enantiomer has been obtained in the hydrolysis, it will be clear that for the preparation of (-)-dropropizine glycidol must be used that is obtained by hydrolysis of the ester remaining after the enantiospecific enzymatic hydrolysis. In this last case it is not necessary to first hydrolyse the remaining ester. It is also possible to cause such an ester to react with 1-phenylpiperazine in a lower alcohol such as methanol. In this case the acid radical is removed and at the same time glycidol is coupled to 1-phenylpiperazine. It is even possible to couple 1-phenylpiperazine with glycidyl ester without the presence of a lower alcohol, after which the ester group can be removed in a known manner.

For the enzymatic hydrolysis of a glycidyl ester use is made of an ester of an aliphatic or aromatic carboxylic acid and preferably a glycidyl ester of an aliphatic carboxylic acid with 1-18 carbon atoms. Most preferably, use is made of glycidyl butyrate. Glycidyl butyrate can easily be prepared, for example from glycidol and butyryl chloride.

When in the description of the invention below reference is made to the preparation and the conversion of glycidyl butyrate, this is done for reasons of simplicity and other glycidyl esters must be regarded as also being included.

Enzymatic hydrolysis of esters is generally known and many different enzymes may be used for this purpose, in particular hydrolases, for example esterases such as pig's liver esterase, pancreatin, lipases, for example from pig's pancreas extract, cholinesterases, proteolytic enzymes, etc. Proteolytic enzymes are protein-splitting enzymes which can be isolated from, for example, different kinds of bacteria such as Bacillus species. They are produced on a technical scale, are commercially available and are capable of hydrolysing esters. Lipases are fat-hydrolysing enzymes. They can be obtained from bacteria, but can also be obtained from yeast and/or fungus species and from animal organs such as pig's liver, pig's pancreas, etc. Lipases are also produced on a technical scale and are commercially available.

The enantioselective enzymatic hydrolysis can be carried out with any enzyme that effects enantioselective hydrolysis. Usually, an enzyme that results in a good combination of activity, selectivity and costs will be used. Lipase and in particular pig's pancreas lipase is a good choice.

The reaction conditions of the hydrolysis are not of critical importance. The enzyme is usually active at the water-organic solvent interface. Usually, the hydrolysis is carried out in water, but mixtures of water and organic solvents that can be mixed therewith may also be used. The glycidyl ester need not be soluble in water and therefore it is not usually necessary to use organic solvents. The pH is set at a value within the activity range of the enzyme. Usually, the hydrolysis is carried out at ambient temperature, but lowered temperatures of -10¤ to -20¤C or slightly elevated temperatures up to about 60¤C may also be used.

Complete hydrolysis of the racemic ester mixture leads to racemic glycidol. Therefore, the hydrolysis must remain incomplete. The hydrolysis of the glycidyl ester is carried out in such a manner that after addition of a buffer and mixing with the enzyme, the pH is maintained at a constant value within the activity range of the enzyme by titration with a base. The amount of base consumes is a measure of the progress of the hydrolysis. Once the desired degree of hydrolysis has been reached, the hydrolysis is stopped and the unconverted glycidyl ester is extracted with a suitable solvent that does not mix with water.

The enantiomeric excess in the glycidol formed in hydrolysis decreases as the conversion proceeds. By hydrolysing for a short period of time and maintaining a low degree of conversion greatly enantiomer-enriched glycidol is obtained, but this is at the expense of the yield. Longer hydrolysis and a higher degree of conversion lead to a higher yield, but then the glycidol is less enantiomer-enriched. In the case of the other glycidol enantiomer, the unhydrolysed ester must be recovered, which contains an excess of the enantiomer that is present in the hydrolysed glycidol in a minor amount, and glycidol must be recovered from this residual ester by hydrolysis. With the unhydrolysed ester the enantiomeric excess increases as the hydrolysis proceeds, but this is again at the expense of the yield. For a person skilled in the art it will be easy to choose the optimum embodiment.

From the above it will be clear that the enantiomeric excess of glycidol is not of very critical importance for the coupling with 1-phenylpiperazine into dropropizine and the recovery of optically pure dropropizine therefrom and may vary within very wide limits. Usually the 1-phenylpiperazine will be coupled with the glycidol formed in the hydrolysis, because with most enantiospecific enzymatic hydrolyses this glycidol contains an excess of the (+)-isomer and is therefore suitable for the preparation of (-)-dropropizine. This coupling is usually done in water. The dropropizine can be recovered from the aqueous solution by cooling the solution, which causes the dropropizine to precipitate, but also a very suitable method is to extract the dropropizine with an organic solvent that cannot be mixed with water and recover it therefrom by cooling and/or evaporation or by addition of a liquid in which the product is poorly soluble. Then the dropropizine enantiomer can be purified by recrystallization from a suitable solvent such as acetone, dichloromethane, alcohol, etc., or a mixture of solvents. Usually two crystallizations are sufficient to raise the enantiomeric excess to over 99%.

The invention will be further elucidated with the following example.

### Example I

### a) Preparation of glycidol with an enantiomeric excess.

250 g of racemic glycidyl butyrate (1.74 moles) was mixed with 250 ml of TRIS-HCl buffer (TRIS = Tris(hydroxymethyl) aminomethane; 50 mM, pH = 7.8) in a 1 l flask. 6 g of pig's pancreas lipase was added, with stirring, the stirring was continued and the butyric acid that was formed was neutralized by automatic titration with 10 M NaOH, the pH being maintained at 7.8. After 78 ml of 10 M NaOH had been added (4 hours; 0.78 mole; 45% conversion) the unconverted (-)-glycidylbutyrate was extracted twice, each time using 500 ml of dichloromethane. The aqueous phase containing an excess of (+)-glycidol was used to prepare (-)-dropropizine.

### b) Coupling with 1-phenylpiperazine.

120.5 g of 1-phenylpiperazine (0.74 mole; 0.95 eq.) was added to the aqueous phase, with stirring, which resulted in an exothermic reaction. The stirring was continued for 2 more hours at 50¤C. This was followed by cooling to ambient temperature and the (-)-dropropizine was twice extracted from the aqueous phase, with 500 ml of dichloromethane. The dichloromethane solution was evaporated until dry, yielding 168 g of impure (-)-dropropizine.
[α]²⁰_{D} = 18.2¤ (c = 3, 1 n HCl).
[α]²⁰_{D} = 15.3¤ (c = 3, CHCl₃).
The impure (-)-dropropizine was recrystallized twice from acetone, yielding 86 g of (-)-dropropizine.
[α]²⁰_{D} = -30.8¤ (c = 3, 1 n HCl);
[α]²⁰_{D} = -25.9¤ (c = 3, CHCl3), melting temperature 103.3-104.6¤C. After a third recrystallization from acetone, these physical properties remain unaffected.

### Example II

40 gram mixtures of crystalline optically pure (-)-dropropizine and racemic dropropizine were prepared as indicated in table 1, which mixtures have enantiomeric excesses of (-)-dropropizine from 20 to 90%.
Each of the mixtures was brought into a 250 ml flask and dissolved by heating in 200 ml of acetone. By cooling the resulting solutions dropropizine crystals were obtained in an amount as indicated in table 1. The optical rotations [α]²⁰_{D} (c = 3, 1 n HCl) were determined and thus the enantiomeric excesses after crystallization were found. The figures are presented below.

**TABLE 1**

| Starting material | | | After crystallization | | | |
|---|---|---|---|---|---|---|
| (-)-dropropizine (gram) | (±)-dropropizine (gram) | e.e. (%) | dropropizine | | | |
| | | | (gram) | (% yield) | [α]²⁰_{D} | e.e. (%) |
| 8 | 32 | 20 | 29.5 | 73.8 | - 4.8 | 15.6 |
| 12 | 28 | 30 | 30.2 | 75.5 | - 9.4 | 30.5 |
| 16 | 24 | 40 | 29.3 | 73.3 | - 13.4 | 42.9 |
| 20 | 20 | 50 | 28.8 | 72.0 | - 17.5 | 56.8 |
| 24 | 16 | 60 | 29.8 | 74.5 | - 22.5 | 73.1 |
| 28 | 12 | 70 | 27.4 | 68.5 | - 28.8 | 93.5 |
| 32 | 8 | 80 | 28.3 | 70.8 | - 30.3 | 98.4 |
| 36 | 4 | 90 | 31.3 | 78.3 | - 30.8 | 100 |

### Example III

### Preparation of (+)-dropropizine

To a reaction mixture obtained by mixing 28.8 g of R(-)-glycidylbutyrate with an enantiomeric excess of 93% with 120 ml methanol and 0.4 gram benzyltriethylammoniumchloride in a 250 ml flask, and heating the mixture during 2 hours at 50¤C, 32.4 g of 1-phenylpiperazine (0.2 mole) was added at room temperature, with stirring, which resulted in an exothermic reaction. The stirring was continued for 1 more hour at reflux temperature (66¤C). The reaction mixture was evaporated until dry, yielding 48 g of impure (+)-dropropizine.
The impure (+)-dropropizine was recrystallized from 250 ml acetone, yielding 36.5 g (77%) of (+)-dropropizine.
[α]²⁰_{D} = -30.4¤ (c = 3, 1 n HCl), melting temperature 102.7-104.5¤C, corresponding with an increased e.e. value of about 99%.

The results of these Examples clearly show that dropropizine with large enantiomeric excess can be obtained by coupling 1-phenylpiperazine with glycidol containing an excess of one of the enantiomers, and crystallization of the obtained coupling product. Eventually 100% optically pure dropropozine can be obtained by repeated crystallization.

## Claims

1. Process for the preparation of enantiomers of dropropizine [3-(4-phenyl-1-piperazinyl)-1,2-propanediol], characterized in that 1-phenylpiperazine is in a manner known per se coupled with glycidol containing an excess of the enantiomers of glycidol and the dropropizine containing an excess of one of the enantiomers of the dropropizine obtained as coupling product is purified by recrystallization.

2. Process according to claim 1, characterized in that glycidol with an enantiomeric excess of at least 30% is used.

3. Process according to claim 1, characterized in that glycidol with and enantiomeric excess of at most 95% is used.

4. Process according to any one of claims 1-3, characterized in that glycidol is used, which has been obtained by means of enantioselective enzymatic hydrolysis of a glycidyl ester.

5. Process according to claim 4, characterized in that glycidol is used, which has been obtained by means of an enantioselective enzymatic hydrolysis of a glycidyl ester with lipase.

6. Process according to claim 4 or 5, characterized in that the glycidyl ester is glycidyl butyrate.

7. Process according to any one of the preceding claims, characterized in that (-)-dropropizine is prepared from glycidol containing an excess of the glycidol (+)-enantiomer.

## Patentansprüche

1. Verfahren zur Herstellung von Enantiomeren von Dropropizin [3-(4-Phenyl-1-piperazinyl)-1, 2-propandiol], dadurch gekennzeichnet daß 1-Phenylpiperazin auf an sich bekannte Weise mit Glycidol, das einen Überschuß eines der Enantiomere von Glycidol enthält, gekoppelt wird, und das Dropropizin, das einen Überschuß eines der Enantiomere vom als Kopplungsprodukt erhaltenen Dropropizin enthält, durch Umkristallisation gereinigt wird.

2. Verfahren nach Anspruch 1, dadurch gekennzeichnet, daß Glycidol mit einem enantiomeren Überschuß von zumindest 30 % verwendet wird.

3. Verfahren nach Anspruch 1, dadurch gekennzeichnet, daß Glycidol mit einem enantiomeren Überschuß von höchstens 95 % verwendet wird.

4. Verfahren nach einem der Ansprüche 1 bis 3, dadurch gekennzeichnet, daß Glycidol verwendet wird, das mittels enantioselektiver enzymatischer Hydrolyse eines Glycidylesters erhalten wurde.

5. Verfahren nach Anspruch 4, dadurch gekennzeichnet, daß Glycidol verwendet wird, das mittels einer enantioselektiven enzymatischen Hydrolyse eines Glycidylesters mit Lipase erhalten wurde.

6. Verfahren nach Anspruch 4 oder 5, dadurch gekennzeichnet, daß der Glycidylester Glycidylbutyrat ist.

7. Verfahren nach einem der vorhergehenden Ansprüche, dadurch gekennzeichnet, daß (-)-Dropropizin aus Glycidol, das einen Überschuß des Glycidol-(+)-Enantiomers enthält, hergestellt wird.

## Revendications

1. Procédé de préparation des énantiomères de dropropizine [3-(4-phényl-1-pipérazinyl)-1,2-propanediol], caractérisé en ce qu'on couple de la 1-phénylpipérazine de manière déjà connue avec du glycidol contenant un excès de l'un des énantiomères de glycidol et on purifie par recristallisation la dropropizine contenant un excès de l'un des énantiomères de dropropizine obtenus comme produit de couplage.

2. Procédé selon la revendication 1, caractérisé en ce qu'on utilise du glycidol avec un excès énantiomorphe d'au moins 30%.

3. Procédé selon la revendication 1, caractérisé en ce qu'on utilise du glycidol avec un excès énantiomorphe de 95% au plus.

4. Procédé selon l'une quelconque des revendications 1-3, caractérisé en ce qu'on utilise du glycidol qu'on a obtenu au moyen de l'hydrolyse enzymatique énantiosélective d'un ester glycidylique.

5. Procédé selon la revendication 4, caractérisé en ce qu'on utilise du glycidol qu'on a obtenu au moyen d'une hydrolyse enzymatique énantiosélective avec une lipase d'un ester glycidylique.

6. Procédé selon la revendication 4 ou 5, caractérisé en ce que l'ester glycidylique est le butyrate de glycidyle.

7. Procédé selon l'une quelconque des revendications précédentes, caractérisé en ce qu'on prépare la (-)-dropropizine à partir de glycidol contenant un excès d'énantiomère (+) de glycidol.
